# EUROPEAN PATENT SPECIFICATION

(11) **EP 1 203 957 B1**
(45) Date of publication and mention of the grant of the patent: **29.11.2006**
(21) Application number: 01309145.9
(22) Date of filing: 29.10.2001
(51) Int. Cl.: G01N 33/543, G01N 33/94

(54) **Multi-analyte immunoassay**
Mehranalyte-Immunoassay
Immunoessai pour les analytes multiples

(30) Priority: 06.11.2000 GB 0027064
(43) Date of publication of application: 08.05.2002
(73) Proprietor: Randox Laboratories Ltd., Crumlin, Co. Antrim BT29 4QY (GB)
(72) Inventor: Fitzgerald, Stephen Peter, Crumlin, Co. Antrim BT29 4QY (GB); Lamont, John Victor, Crumlin, Co. Antrim BT29 4QY (GB); McConnell, Robert Ivan, Crumlin, Co. Antrim BT29 4QY (GB)
(74) Representative: Perry, Robert Edward

(56) References cited:
- EP-A- 0 572 845
- EP-A- 0 617 285
- EP-A- 0 726 464
- WO-A-98/26644
- US-A- 4 595 661
- US-A- 4 722 889
- US-A- 4 743 542
- US-A- 5 026 653

## Description

### Field of the Invention

This invention relates to a multi-analyte immunoassay, e.g. using a biochip or microchip having an array of analyte-specific ligands thereon.

### Background of the Invention

Biochips/microchips have been described for the measurement of large arrays of either immunoassays or nucleotide hybridisation assays. The fabricated arrays are usually designed with numerous individual reaction sites located in spatially-distinct areas on a solid support. The arrays can comprise high densities of the same or different molecules immobilised on the solid support surface.

Whenever a multi-analyte panel is to be tested, it is necessary to correctly quantify the amount of each analyte present in an unknown sample. Therefore, an optimum standard curve must be generated for each analyte represented on the multi-analyte panel. This standard curve must cover a dynamic range suitable for the measurement of the particular analyte it represents, and be suitable for the type of patient sample that is to be measured. This dynamic range is a measure of the sensitivity of the assay to the analyte being measured.

Difficulties arise in the range of concentrations that must be detected for each analyte. As the required concentration range to be measured can be very different for each analyte on the panel, optimisation of each standard curve can present sensitivity problems.

This variety of dynamic ranges that must be encompassed in the standard curves of the multi-analyte panel can lead to practical problems in the production of the multi-conjugate that must be added to each biochip. This multi-conjugate is the reagent by which a detectable marker, appropriate to each individual immunoassay, is added to the biochip. This detectable marker is how each immunoassay is visualised and subsequently quantified on the biochip.

By way of example, it is necessary to control the shape of the standard curve for an array of ligands binding drugs of abuse in a biochip format. The current screening guidelines cover a wide range of concentration; for example, the cut-off range is from 0.5 ng/ml for LSD to 2000 ng/ml for opiates.

This is quite challenging to achieve, given that the molecules in question are largely quite similar in terms of their molecular weight and immunogenicity. Therefore all antibodies to these molecules have quite similar characteristics in terms of detection limits achievable. In current testing methods, each drug has its own specific test. This permits the test manufacturer to modify each individual test to best accommodate the screening guideline for that particular drug test. However, in a multianalyte format, the conditions are applied to the complete range of assays present on the biochip. Therefore the options available to accommodate a wide range of cut-off limits are limited.

US-A-4722889 and US-A-5026653 disclose assaying for an analyte in a sample by reaction with a labelled antibody having specificity for the analyte. In addition, a scavenger monoclonal antibody may be used, having low specificity for the analyte but high specificity for analogues that act as interferents.

US5026653, US4595661, WO98-26644, EP0572845 and EP0617285 disclose immunoassays for analytes, wherein the samples are contacted with a ligand specific for the analyte and additionally with a scavenger antibody that binds the analyte.

WO-A-98/26644 discloses an assay for an analyte, e.g. a drug of abuse, in a sample, using a "detecting antibody" that reacts with analyte. A second reaction uses a "neutralising antibody" (that may be the same as the detecting antibody) that binds analyte and thereby prevents it from giving a positive reaction in an assay conducted with a detecting antibody. Thus, samples giving a positive reaction with the detecting antibody but decreased reaction in the second reaction contain the true analyte; samples giving similar positive reactions in both contain an interferent. An advantage of this procedure is that it avoids the need to know the precise nature of the interferent.

### Summary of the Invention

The present invention is based on the realisation that the optimisation of the dynamic range can be difficult when it is considered that each analyte must be detected at often very different concentrations to the others on the panel and that each conjugate must be represented in the multi-conjugate reagent at a concentration/dilution appropriate to the particular analyte it recognises. It has now been appreciated that the analytes within the panel may need to be detected and quantified at very different concentrations from each other in a biochip.

Specifically, the invention relates to altering the sensitivity of a standard curve which is found to be too sensitive in one region and not sensitive enough in another region, for an analyte/range of analytes found in the multi-analyte panel under consideration. It involves the addition of a certain amount of a scavenger antibody, or other scavenger material, to the assay system, e.g. to the assay buffer. This scavenger material may or may not be the same as that which is immobilised on the biochip surface to capture the analyte. The purpose of the scavenger material is to specifically scavenge/mop-up some of the analyte in the sample (standard or patient sample) and therefore reduce its available measurable concentration. This in turn has the effect of reducing the sensitivity of a very sensitive portion of a standard curve and improving the sensitivity of another region of the graph.

### Description of the Invention

A biochip for use in the invention can be produced by known procedures. See, for example, GB-A-2324866.

Such a biochip presents an array of ligands, e.g. antibodies. There may be several different ligands having specificity for different analytes. The novel assay can be used to test for one or more such analytes, and may involve the use of one or more antibodies. Other scavenging materials that may be used will be readily apparent to the skilled person, and include aptamers. The scavenger material will typically be included in the assay buffer which otherwise usually contains conventional components such as solvents etc.

The sensitivity of a standard curve is a function of B/Bo, wherein B and BO respectively represent the light unit values for bound species and zero standard. A standard curve may be constructed in each case where a quantitative assay is required. Alternatively, e.g. for drugs of abuse assays, a yes/no assay is satisfactory, for which purpose calibration at 100% cut-off is appropriate.

For the purpose of illustration, the invention will be considered in relation to a drugs of abuse multi-analyte panel, and using an antibody as the scavenger. A typical such panel comprises competitive immunoassays for the following drugs of abuse: amphetamine, methamphetamine, THC, benzodiazepine, opiates, barbiturates, benzoylecgonine, methadone, PCP and LSD.

For the drugs of abuse standard curve (0% - 300%), the 100% standard contains the cut-off concentration of each of these drugs, i.e. the concentration below which the sample is declared to be negative for the drug and above which the sample is declared to be positive for the drug. It is the wide range of cut-off concentrations, as listed below, that causes problems in obtaining suitable standard curves for all analytes from the addition of a multi-conjugate.

| Drug of Abuse | Cut-off |
|---|---|
| Amphetamine | 1000 ng/ml |
| Methamphetamine | 1000 ng/ml |
| THC | 50 ng/ml |
| Benzodiazepine | 200 ng/ml |
| Opiates | 2000 ng/ml or 300 ng/ml |
| Barbiturates | 200 ng/ml |
| Benzoylecgonine | 300 ng/ml |
| Methadone | 300 ng/ml |
| PCP | 25 ng/ml |
| LSD | 0.5ng/ml |

In determining the amount of scavenger antibody to be used, a compromise must generally be made between increasing the value of B/Bo between the 0% and 25% standards and not reducing the drop between 75% - 100%-125%. In other words, although the aim is to make the curve less sensitive between 0% and 25%, it must not become less sensitive between 75% and 125% where cut-off decisions for each drug will be made. It is therefore important to maximise the sensitivity around the cut-off region so that accurate determinations of the positivity or negativity of a sample can be made.

The cut-off region for any particular analyte is known or can readily be determined by a person of ordinary skill in the art. Such a person can also readily determine how the sensitivity of the curve may be maximised at around this point.

Experiments have been conducted, using PCP, BZG and amphetamine, to demonstrate the degree of alteration achieved, at the cut-off region, of standard curves, by the addition of different amounts of the appropriate scavenging antibody. More particularly, the following data for PCP, BZG and amphetamine demonstrate that, without the addition of scavenger antibody, the standard curve is not sensitive enough at the cut-off region (in each case, it was too sensitive between 0% and 25%). For the purposes of this invention, and these analytes, the curves are too sensitive between 0% and 25% and not sensitive enough beyond that point.

The results for PCP are as follows:

**Table 1**

| **Standard** | **B/Bo 0 Ab** | **B/Bo 0.5 Ab** | **B/Bo 1 Ab** | **B/Bo 2 Ab** |
|---|---|---|---|---|
| 25 | 69.37 | 94.88 | 113 | 108.3 |
| 75 | 31.79 | 44.61 | 69.78 | 95.72 |
| 100 | 25.65 | 32.59 | 52.18 | 64.49 |
| 125 | 20.34 | 26.58 | 38.4 | 49.5 |

The addition of 1-2 mg/L PCP antibody considerably improves the sensitivity of the curve around the cut-off region of 75 - 125%. This is clearly demonstrated in the percentage inhibition drop from 11.45% (B/Bo 31.79 at 75% to 20.34 at 125%) with no antibody added, to 46.22% (B/Bo 95.72 at 75% to 49.50 at 125%) with the addition of 2mg/L PCP antibody.

The results for BZG are as follows:

**Table 2**

| **Standard** | **B/Bo 0 Ab** | **B/Bo 20 Ab** | **B/Bo 50 Ab** | **B/Bo 80 Ab** |
|---|---|---|---|---|
| 25 | 31.77 | 43.46 | 73.41 | 76.14 |
| 75 | 14.46 | 19.54 | 32.45 | 47.89 |
| 100 | 12.14 | 17.81 | 27.44 | 40.23 |
| 125 | 9.47 | 13.03 | 20.38 | 30.38 |

A substantial amount of BZG antibody must be added to significantly alter the sensitivity of this standard curve. Between 50 and 80 mg/L must be added to improve the sensitivity of the cut-off region. With no antibody added, the percentage inhibition drop is 4.99% (B/Bo 14.46 at 75% to 9.47 at 125%), and this improves to 17.51 % (B/Bo 47.89 at 75% to 30.38 at 125%) with the addition of 80 mg/L BZG antibody.

The results for amphetamine are as follows:

**Table 3**

| **Standard** | **B/Bo 0 Ab** | **B/Bo 0.5 Ab** | **B/Bo 1.25 Ab** |
|---|---|---|---|
| 25 | 14.46 | 76.52 | 98.55 |
| 75 | 5.61 | 38.73 | 67.83 |
| 100 | 4.32 | 22.57 | 43.58 |
| 125 | 3.49 | 20.16 | 40.91 |

1.25 mg/L amphetamine antibody was the optimum addition to improve the sensitivity of the cut-off region of the standard curve. Concentrations above 1.25 mg/L did not improve the result further. With no antibody added, the percentage inhibition drop is 2.12% (B/Bo 5.61 % at 75% to 3.49% at 125%), and this improves to 26.92% (B/Bo 67.83% at 75% to 40.91% at 125%) with the addition of 1.25 mg/L amphetamine antibody.

## Claims

1. An assay for analytes in a sample, wherein the sample is contacted with a biochip presenting an array of ligands each specific for an analyte, and which additionally comprises including a scavenger material that binds an analyte and thereby reduces its available concentration.

2. An assay according to claim 1, wherein the analytes are drugs of abuse.

3. An assay according to any preceding claim, wherein the scavenger material is an antibody.

4. An assay according to claims 1 to 3, wherein the amount of the antibody that is added maximises the sensitivity of a standard curve at around the cut-off region.

## Patentansprüche

1. Assay für Analyten in einer Probe, in dem die Probe mit einem Biochip in Kontakt gebracht wird, der eine Matrix von Liganden darbietet, die jeweils spezifisch für einen Analyten sind, und der zusätzlich umfasst, dass ein Abfängermaterial eingeschlossen ist, das einen Analyten bindet und **dadurch** dessen verfügbare Konzentration verringert.

2. Assay nach Anspruch 1, in dem die Analyten Missbrauchs-Drogen sind.

3. Assay nach irgendeinem vorangehenden Anspruch, in dem das Abfängermaterial ein Antikörper ist.

4. Assay nach den Ansprüchen 1 bis 3, in dem die Menge des Antikörpers, die zugesetzt wird, die Empfindlichkeit einer Standardkurve entlang des Ausschlussgrenzenbereichs maximiert.

## Revendications

1. Test pour déterminer des substances à analyser dans un échantillon, où l'échantillon est mis en contact avec une biopuce comportant un réseau de ligands, chacun spécifique d'une substance à analyser, et qui comprend en outre l'inclusion d'une matière piège, qui se lie à une substance à analyser et en réduit ainsi la concentration disponible.

2. Test selon la revendication 1, où les substances à analyser sont des drogues.

3. Test selon l'une quelconque des revendications précédentes, où la matière piège est un anticorps.

4. Test selon les revendications 1 à 3, où la quantité d'anticorps qui est ajoutée rend maximale la sensibilité d'une courbe d'étalonnage autour de la région du seuil de positivité.
